# EUROPEAN PATENT APPLICATION

(11) **EP 1 262 902 A1**
(43) Date of publication of application: **04.12.2002**
(21) Application number: 02290281.1
(22) Date of filing: 06.02.2002
(51) Int. Cl.: G06F 19/00

(54) **System and method to assist a dentist or dental surgeon in performing dental procedures**

(30) Priority: 30.05.2001 FR 0107091
(71) Applicant: Vannoye, Michel, 78860 Saint Nom la Bretèche (FR)
(72) Inventor: Vannoye, Michel, 78860 Saint Nom la Bretèche (FR)
(74) Representative: Fruchard, Guy

(57) **Abstract**

A system for providing step-by-step assistance to dentists or dental surgeons in following dental protocols, comprising:
a central server (98) having storage (102) including a plurality of image presentations relating to different dental protocols;
a plurality of satellite installations for location in individual dental offices (82, 94, 96), with the dental offices including the patient dental chair or chaise (84) with the usual dental drill (89) and other dental equipment, each said satellite installation including a video monitor (90), a computer and local storage (92); with the monitor being mounted adjacent the dental chair (84) for viewing by the dental surgeon, while operating on the patient;
means (106) for coupling each said satellite office to said central server (98) for receiving up-to-date image presentations relative to said dental protocols;
said system including controls operable from said satellite installations for displaying selected protocols and displaying images to guide the dentist on a step-by-step basis through the dental protocol; and
said satellite installation (82, 94, 96) further including controls for providing in-depth presentations relative to the successive images presented on the video monitor (90), concerning equipment to be used, medications and other products to be employed in the course of the protocol.

## Description

The present invention concerns the facilitation of the work of dental surgeons when they perform dental operations.

A dental surgeon accomplishes a diversity of dental operations such as the preparation of dental molds, the insertion of an implant, etc., which require the knowledge on the part of the dental surgeon of a great number of diverse and varied operating procedures. Those operating procedures can be very detailed and relatively long, which involves either considerable efforts of memorization on the part of the dentist, or of researching the documentation for instance with regard to operations he does not often undertake. Indeed, the practitioner cannot take the risk to commit any error in accomplishing a dental operation in order to guarantee an optimal service for the greatest safety of the patient.

Additionally, the evolution of dental techniques and materials engenders an evolution in operating procedures which the dental surgeon must know in order to be able to practice them. Consequently, the dental surgeon must follow an important number of educational programs in order to stay correct with the evolutions of clinical protocols related to the various dental operations. Those educational programs offered at conventions or by knowledgeable enterprises, turn out to be extremely costly and involve repeated shut downs of the practitioner's dental office, which in turn brings about a significant drop in revenues.

Within this context, the invention makes it possible to overcome the inconveniences presented in the prior art by offering a method as well as means of assistance for guiding the dental surgeon during each dental operation, making it possible for him to not have to memorize the details of each clinical protocol, or to seek documentation before undertaking the dental operation.

To accomplish that objective, in accordance with the invention, the method of providing assistance to a dental surgeon while performing a dental operation, comprises the stages of:
(a) Establishment of a list of the various dental operations which are likely to be performed by the dental surgeon;
(b) Association, with each dental operation, of a clinical protocol or procedure which is to be followed;
(c) Displaying, upon request of the dental surgeon, the said clinical protocol.

According to a first form of implementation, the display of the clinical protocol is accomplished by means of a sequence of stages shown image-by-image on a video monitor.

According to a second form of implementation, the display of the clinical protocol is accomplished by means of a dynamic animation.

Furthermore, the process includes a program for displaying pharmaceutical products through visual identifiers specific to each product, preferably using a video monitor.

Preferably, the process is put into operation by means of a computer, whereby the clinical protocol related to each different dental operation, as well as the pharmaceutical products are automatically updated through connection to an electronic data network, and to a master or central server.

The invention also covers means intended for assisting a dental surgeon during the performance of a dental operation, which is characterized in that it comprises means enabling it to:
(a) establish a listing of various dental operations likely to be performed by the dental surgeon;
(b) associate, with each dental operation, the clinical protocol to be followed;
(c) display, upon demand or selection by the dental surgeon, the said clinical protocol on a video monitor.

In an advantageous way, the system involves the displaying of the clinical protocol by means of a series of sequential steps shown image by image and/or by means of a dynamic animation.

According to a preferred embodiment, the system also comprises, in addition, means for displaying pharmaceutical products including presentation on a video monitor of packaging associated with each product.

In a particular form of implementation, the program and video display may involve:
(a) the establishment of a list of different dental operations;
(b) the association, with each dental operation, of a clinical protocol to be followed:
(c) displaying, upon demand or selection by the dental surgeon, of the said clinical protocol;

The system involves the automatic updating of the clinical protocol related to the various dental operations, and may also involve updating the latest pharmaceutical products when connected to an electronic data network.

In a specific illustrative embodiment of the invention, a system for providing step-by-step assistance to dentists includes the following:
1. a central server having storage including a plurality of video presentations relating to dental protocols;
2. a plurality of satellite installations for location in individual dental offices, with each satellite installation including a video monitor, a micro-processor and local storage;
3. electronic arrangements for coupling each said satellite office to the central server for receiving current video presentations relative to selected dental protocols;
4. the system including controls operable from the satellite installation for displaying selected protocols and displaying video images to guide the dentist on a step-by-step basis through the dental protocol; and
5. the satellite installation further including controls for providing in-depth presentations relative to the successive images presented on the video monitor, concerning equipment to be used, medications and other products to be employed in the course of the protocol.

The invention can be better understood from the detailed description which follows, which is provided as an example for purposes only of illustration and not of limitation, by reference to the annexed drawings.

Fig. 1 consists of a schematic drawing illustrating the steps of a program which embodies the invention;

Fig. 2 is an enlarged showing of a video monitor screen showing a dental protocol, with an enlarged showing of a particular step in the program.

Fig. 3 shows a program involving the preparation of prescriptions;

Fig. 4 represents a program involving various collateral functions which may be available through the system;

Fig. 5 is a schematic view of the complete system including the central or master server and the satellite installations;

Fig. 6 is an enlarged showing of one of the satellite installations in a dentists office; and

Figs. 7A and 7B together show an overall program which may be made available to the dentist through the present system.

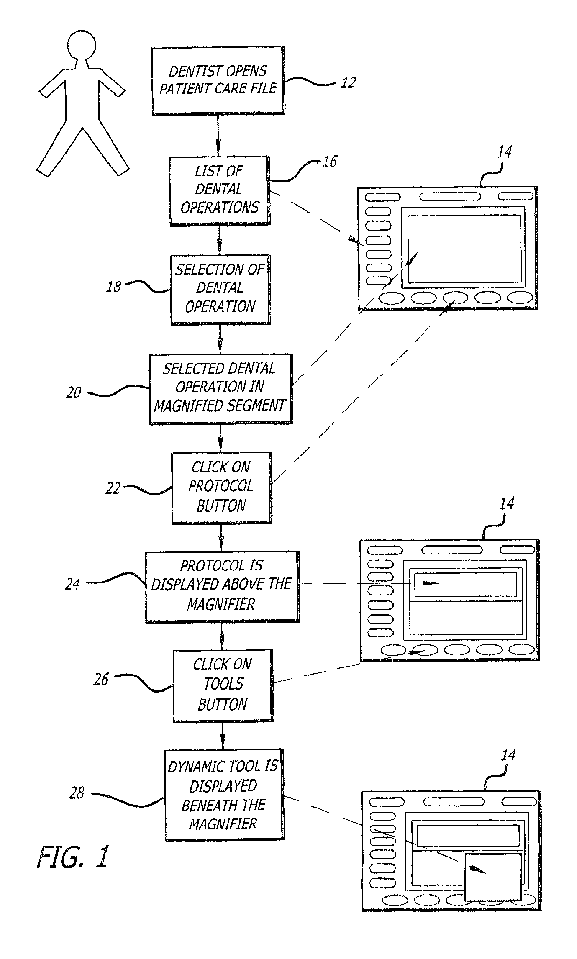

Figure 1 is a schematic layout illustrating different steps of a program which implements the principles of the invention while a dental operation is being performed by a dental surgeon. In accordance with one preferred embodiment, assistance to the dentist is provided by a computer which includes the steps of recording, storing, selecting and displaying information useful to the dentist.

The setup according to the invention includes arrangements for setting up a data base configuring the various dental operations which are likely to be performed by a dental surgeon. The said setup also makes it possible to associate with each dental operation the clinical protocol which must be followed during the performance of the dental operation. As suggested, above, the clinical protocol of a dental operation can turn out to be relatively complex and to contain a great number of very important details.

For instance, in the case of the insertion of an implant, the operating procedure comprises various steps which must be followed, as well as a certain number of criteria which must be taken into account. such criteria may consist for instance of the diameter of the cavity to be drilled, the cavity's depth to be attained, the instruments to be used, etc., and these may differ according to the patient's age, the manufacturer of the implant, and other factors.

The setup also comprises equipment enabling the display of a plurality of data such as the list of the various dental operations, a list of patients, as well as the clinical protocol for the selected dental operation.

Referring to Figure 1, step 12 of the process is a step during which the dental surgeon selects from among the list of his patients, the one on whom the dental operation is to be performed. A video monitor 14 may be employed to display the list of patients, and, using a mouse or similar control, and clicking on the patient's name, the patient's care file is opened, and may be displayed on the monitor. Once that patient's care file is opened, the list of the various operations already performed, or still to be performed is displayed so that the dental surgeon can go through it (step 16).

In the next step 18, the dental surgeon selects the dental act or procedure which he will be performing on the patient. It is advantageous to make this selection of a dental operation to be performed by means of a magnifier or enlargement on the video monitor so that the operation to be performed will stand out over the other operations appearing on the list, see block 20 of the program. Once the operation is selected, the dental surgeon has the possibility, at his request and in real time, to cause clinical protocol related to the selected operation to be displayed on the computer screen, or video monitor, see steps 22 and 24. For that purpose, he may use any of several means of selection (such as, for instance, a button which he can activate by means of a pointing device or mouse) in order to make the clinical protocol appear. Thus, if the dental surgeon wishes, the clinical protocol can be presented to him so that he can follow it while performing the dental operation (step 24 of the process).

The dentist may also receive information regarding the dental instruments or tools to be used in the protocol (block 26) and receive an enlarged image thereof (see step 28).

According to a first form of embodiment, the display of the clinical protocol can be achieved with the help of a sequence of images which illustrate the different steps to be followed to perform the dental operation. Those different images can thus take into account the criteria which must be respected, as well as the order in which the operations are to be performed.

The dynamic display of the clinical protocol can also be obained in parallel with the image by image display in order to provide the dental surgeon the most complete information possible to assist him.

The display of the clinical protocol is adapted to the clinical protocol and depends on its nature. Indeed, in the case where a precisely timed delay is to be respected during an operation, the process covered by the invention provides the display, for example, of a chronometer, thereby making it easy for the dental surgeon to respect delays suggested by the clinical protocol.

There is the advantage that the various protocols can be updated automatically by a connection to a Central or Master Server, via the internet or other coupling the Central server. Thus, as soon as new products make their appearance or become available or modifications to the existing protocols, the setup and the process are immediately updated, and without any additional effort required from the individual practitioner.

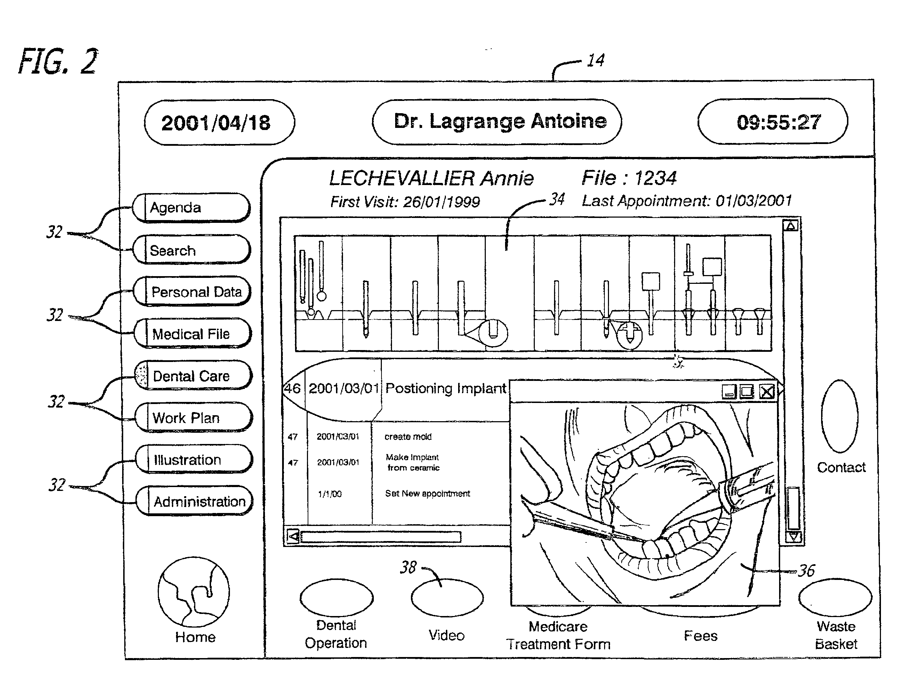

Referring now to Fig. 2 of the drawings, it is an enlarged showing of the monitor 14, with the information on the screen corresponding to that which would be displayed in association with step 24 of the program of Fig,. 1. In Fig. 2, the control points 32 are accessed by a mouse or other comparable method. Following actuation of control point designated "Dental Care", the dentist selects a particular protocol, such as "root canal" or "dental implant", or the like. Then as shown at 34 toward the upper area of the display, the complete protocol for the operation is set forth. Upon "clicking" on, or actuating a particular protocol shown in the area 34, an enlarged or magnified showing 36, appears at the lower portion of the screen. A video presentation of the particular step involved in the operation may be obtained by actuating the control point 38 on the monitor image.

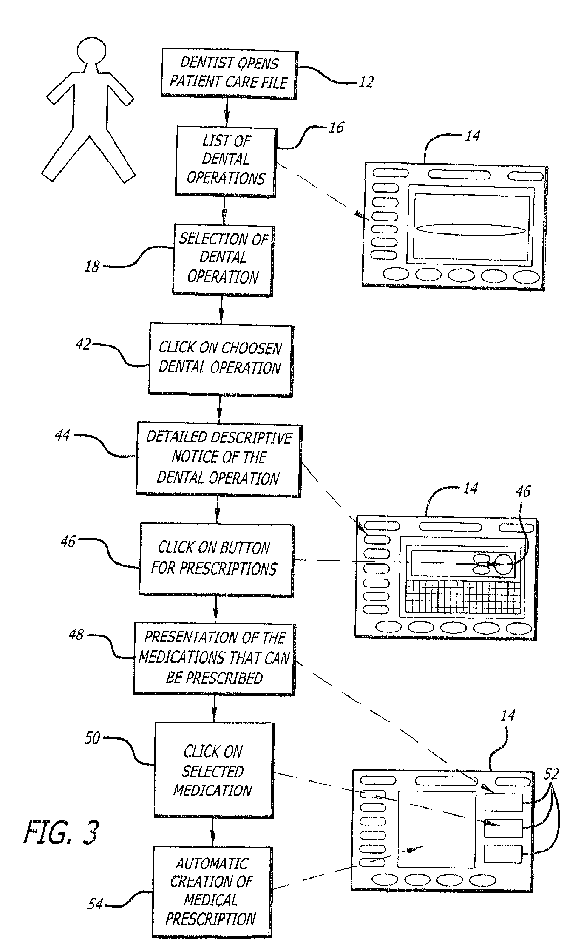

Figure 3 is a schematic drawing illustrating another advantage of the system. The steps 12 for the opening of a patient care file, 16 for displaying the list of dental operations, and 18 for selecting a particular dental operation from among those on the displayed list, are identical to those described in connection with Figure 1. Once a dental operation has been selected pursuant to step 42, the system provides the possibility, at step 44 to select file describing in detail the dental operation so selected in the previous step 42. By the actuation of or "clicking" on a prescription control point 46 on monitor 14, a list of products is displayed which may be prescribed within the context of the performed dental operation (step 50). Such display can be advantageously carried out by means of a visual identifier specific to each product, and in particular through a reproduction of the actual box of the product to be prescribed, indicated by step 50 through which box images 52 are displayed on the monitor 14.

The displaying of the products which may be prescribed, can be setup in accordance with their frequency of prescription whether in a decreasing order, in alphabetical order, or in any other manner. Again, in the same way as was done for the clinical protocols, the program advantageously provides for the automatic updating at a distance of the list of prescription products, for example by means of a connection to an electronic data network such as Internet, or other coupling to the Master or Central Server.

For instance, when a new product comes on the market, it is included in the data base within the Central or Master Server, and upon accessing this Central Server, the dental surgeon becomes immediately informed about that new product as well as about its properties. Once the medication or the different products have been selected by the dental surgeon, possibly in agreement with the patient and in accordance with the indications shown in his dental care file, the automatic generation of the dental prescription for the patient is indicated at program step 54. Once produced and is displayed, the dental prescription can be edited with the help of computer keyboard typing known in the prior art and associated with the computer.

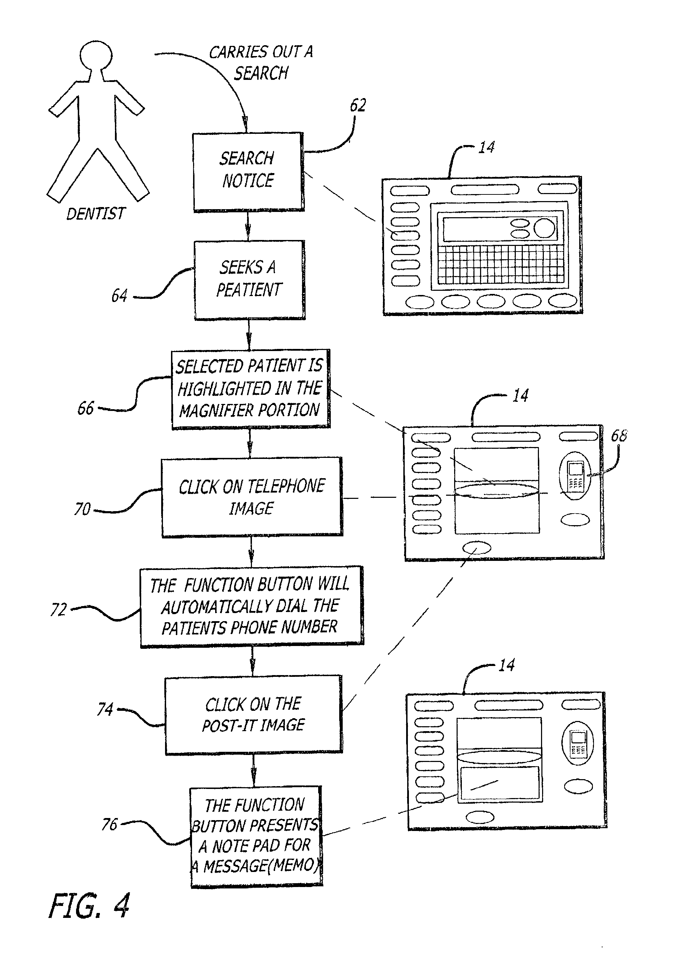

Figure 4 is a schematic drawing illustrating a third program which may be available through the system. The program of Fig. 4, including steps 62, 64 and 66, provides indeed, in connection with step 70, the possibility for displaying the list of the several patients of the dental surgeon, and selecting a specific patient. Once a patient has been selected from that list the file showing the detailed profile of such client appears thanks to a graphic interface. That file comprises advantageously a series of visual identifiers which are destined to facilitate certain activities of the dental surgeon.

A first example of such a visual identifier consists of a visual representation 68 of a commercial telephone. By selecting the said telephone by means of a pointing device or mouse in step 20, the system contains program and circuitry arrangements which are known by themselves and which automatically dial the phone number of the patient (step 72). Other visual identifiers can likewise select certain functions of the setup in,such as a note pad of the "Post-it®" kind for purposes of writing and printing a memo, per steps 74 and 76.

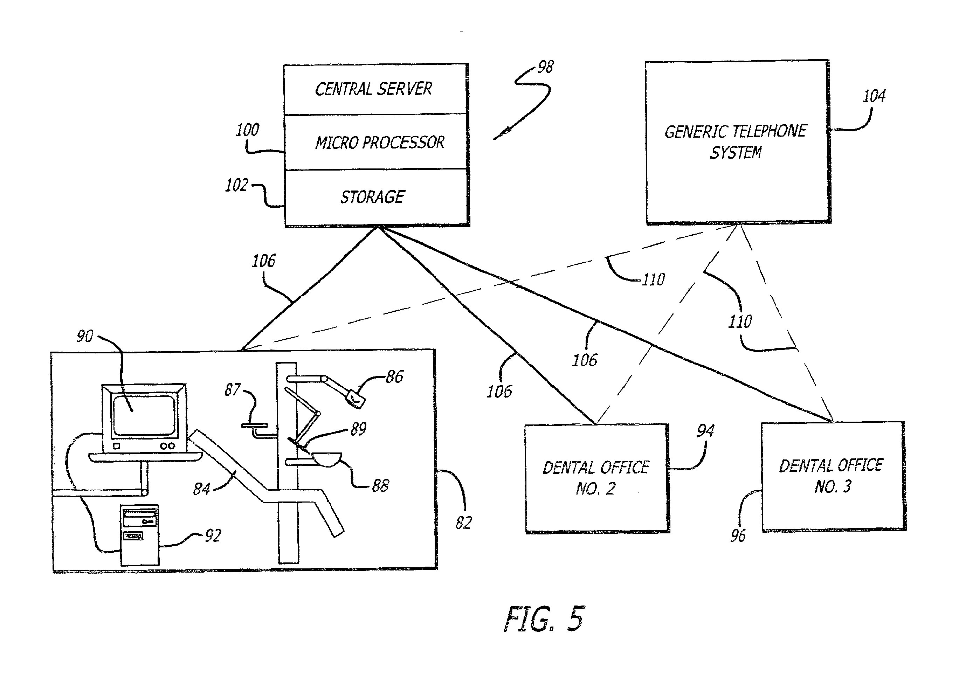

Referring now to Figure 5 of the drawings, a dental office is illustrated at reference numeral 82, with a dental reclining chair or chaise shown at 84; and with the usual equipment of a dental office including a lamp 86, a tool support tray 87, a liquid waste bowl 88, and a drill equipment 89 being shown schematically. In addition, a video monitor 90, and an associated computer terminal 92, are shown in the dental office 82. The computer 92 may include memory, a printer, and an optional keyboard

There are additional dental offices indicated at reference numerals 94 and 96. All of these dental offices, which may be considered to be satellite locations, are coupled to the central server 98, which includes a microprocessor 100, associated storage 102. In addition, a conventional public utility telephone system 104, is coupled in a conventional manner to each of the satellite dental offices, 82, 94, and 96.

The individual dental offices 82, 94, and 96 are coupled to the central server 98 as indicated by the lines bearing reference numeral 106. Incidentally, it is to be understood that the computers such as computer 92 associated with the satellite dental offices, are provided with storage capacity. Accordingly, when a dentist is planning to perform a particular surgical procedure or protocol he may couple his computer video monitor directly via the internet to the central server 98. Alternatively, the lines 106 may be telephone lines and prior to the need to use the particular dental protocol, the dentist may download the selected program(s) from the central server 98 to the local storage associated with his computer, such as computer 92 in dental office 82. The downloaded program will then be available for access at high speeds as needed by the dentist in his local office. Alternatively, through the use of the internet, or otherwise, the satellite offices may have direct links to the central server 98, and receive the needed protocol information directly from the central server 98, without advance local storage on the specific computer 92 in the local office 82.

As mentioned previously in connection with a program of Figure 4 of the drawings, the computer may be coupled to a local telephone line at the dental office to dial customers telephone numbers through the telephone system 104. These connections are indicated diagramatically by the dashed lines 110 which appear in Figure 5.

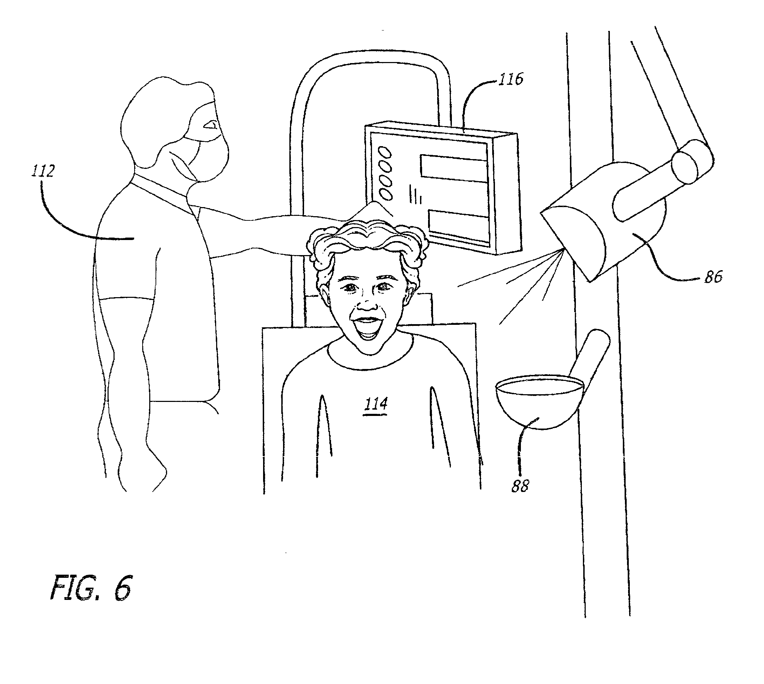

Referring now to Figure 6 of the drawings, the inside of the dental office such as the office 82 as shown in Figure 5, is portrayed as including the dentist 112, a patient 114 and the computer monitor 116. In addition, the intense light fixture 86 and the liquid waste receptacle 88 are shown in this figure of the drawings.

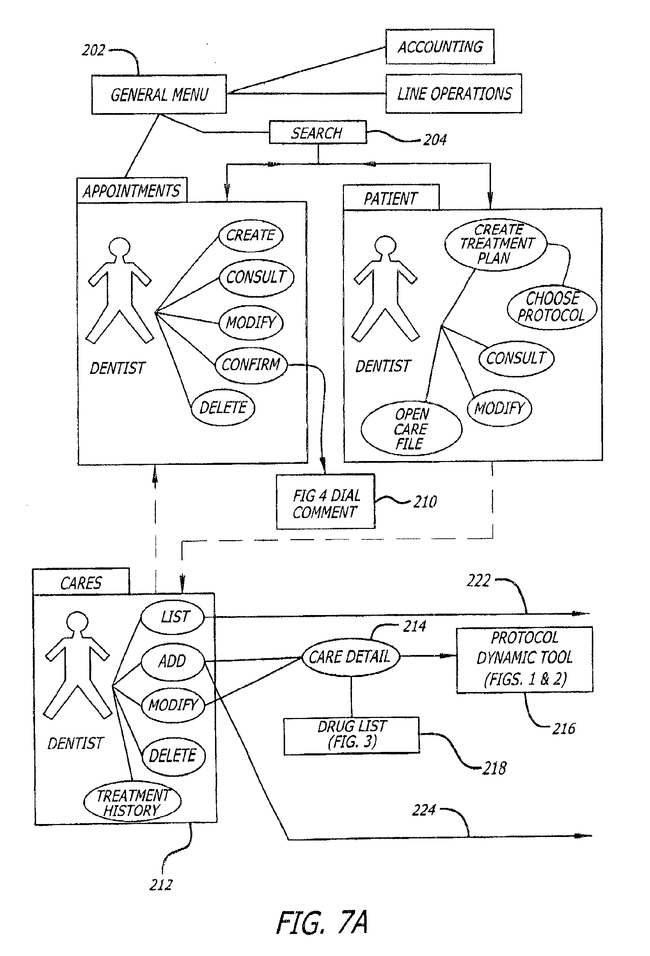

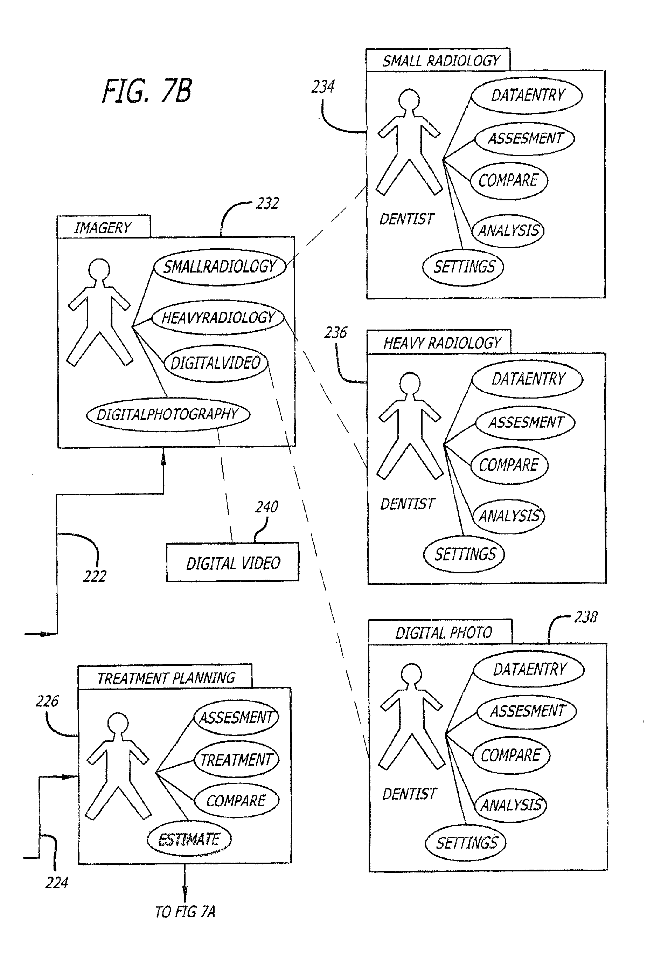

Now, referring to Figures 7A and 7B, they constitute a general menu or general program available with the dentist's computer and video monitor, as shown in Figures 5 and 6 of the drawings. In considering Figures 7A and 7B, starting with the block 202 at the upper right left corner of Figure 7A, the principal program matters with which we are concerned in the present patent application involve the search block 204 which could identify a specific patient, as indicated by the block 206. This step is often a preliminary step in the use of the system of the invention. Once the patient is identified, the system may display images corresponding to Figure 4 of the drawings as indicated by the reference numeral 208 in Figure 7A. Note that block 72 in Figure 4 involves directly dialing the patient through the use of the image on the video monitor once the patient has been identified. This indicated by the block 210 in Figure 7A. Block 212 involves the cares which are planned by the dentist and the patient identified at 206, and includes the steps indicated both in Figures 1 and 2, see reference numerals 214 and 216 in Figure 7A; and they also include the drug list and prescription steps of Figure 3, as indicated by the block 218 in Figure 7A.

Figures 7A and 7B are coupled as indicated by the lines and arrows 222 and 224 which interconnect Figures 7A and 7B. Referring first to block 226 in the lower left hand corner of Figure 7B, this involves treatment planning; following the analysis and estimates, the output from block 226 is routed back to Figure 7A for discussion with and confirmation by the patient, as indicated at 208.

Referring to the input 222 the imagery block 232 in Fig. 7B, various forms of imagery are available to the dentist. This involves two forms of X-ray imagery indicated at blocks 234 and 236 of the drawings, digital video and photography, both still pictures as indicated at blocks 238 and animated or video images as indicated by the block 240. In the implementation of the video imaging, a video camera may be provided; and in the implementation of the radiology functions, a special digital pickup with an array of sensors may be employed, and in both cases the images may be stored or viewed on the monitor.

In conclusion, it is to be understood that the embodiments shown in the drawings and described in detail in the specification are illustrative of the principles of the invention. Various alternatives and modifications may be made without departing from the spirit and scope of the invention. For example, the selected surgical protocol can be down loaded from the central server to the local storage in advance of need, or may be viewed on-line with a direct coupling from the satellite monitor to the central server. In addition, any desired arrangements for coupling the individual dental offices central server may be employed, including fiber optic cables and radio frequency transmissions, by way of example. The local computer at the dentists office may include a printer and a keyboard. The keyboard may be implemented either by a separate physical keyboard or by the image of a keyboard on the monitor 14, with the letters on the keyboard image being accessed either by a mouse, or by using a touch or wand sensitive monitor screen. In addition, other local and industry wide developments may be accessed, in addition to the specific matters shown hereinabove. Accordingly, the present invention is not limited to that precisely as shown in the drawings, and described in detail hereinabove.

Thus the system and the program in accordance with the invention, make it possible to assist the dental surgeon with the various dental operations which he is called upon to perform. Further, the displaying in real time, or contemporaneously, of the clinical protocol covering the dental operation which he effectuates on a patient, allows him to operate much more efficiently and to obtain help on demand which he knows to be reliable, safe and constantly up-to-date.

## Claims

1. A system for providing step-by-step assistance to dentists or dental surgeons in following dental protocols, comprising:
a central server (98) having storage (102) including a plurality of image presentations relating to different dental protocols;
a plurality of satellite installations for location in individual dental offices (82, 94, 96), with the dental offices including the patient dental chair or chaise (84) with the usual dental drill (89) and other dental equipment, each said satellite installation including a video monitor (90), a computer and local storage (92); with the monitor being mounted adjacent the dental chair (84) for viewing by the dental surgeon, while operating on the patient;
means (106) for coupling each said satellite office to said central server (98) for receiving up-to-date image presentations relative to said dental protocols;
said system including controls operable from said satellite installations for displaying selected protocols and displaying images to guide the dentist on a step-by-step basis through the dental protocol; and
said satellite installation (82, 94, 96) further including controls for providing in-depth presentations relative to the successive images presented on the video monitor (90), concerning equipment to be used, medications and other products to be employed in the course of the protocol.

2. A system as defined in claim 1 further comprising program arrangements for selectively providing an enlarged showing (20) of any desired images.

3. A system as defined in claim 1 further comprising program arrangements for providing the images in dynamic animation (24).

4. A system for providing step-by-step assistance to dentists or dental surgeons in following dental protocols, comprising:
a central server (98) having storage including a plurality of image presentations relating to dental protocols (44);
a plurality of satellite installations for location in individual dental offices (82, 94, 96), with the dental offices including the patient dental chair or chaise (84) with the usual dental drill and other dental equipment, each said satellite installation including a video monitor (90), a computer and local storage (92); with the monitor being mounted adjacent the dental chair for viewing by the dental surgeon, while operating on the patient;
means (106) for coupling each said satellite office to said central server (98) for receiving up-to-date image presentations relative to said dental protocols;
said system including controls operable from said satellite installations for displaying selected protocols and displaying images to guide the dentist on a step-by-step basis through the dental protocol; and
said satellite installation (82, 94, 96) further including controls for controlling the timing of and the size of successive images presented on the video monitor.

5. A system as defined in claim 4 further comprising program arrangements for selectively providing an enlarged showing (20) of any desired images.

6. A system as defined in claim 4 further comprising program arrangements for providing the images in dynamic animation (24).

7. A system as defined in claim 4 wherein, following the selection of a dental protocol by the dentist, arrangements are provided for displaying a series of relative small images of the successive steps in the protocol; and further arrangements are provided for providing an enlarged image of any said successive steps as selected by the dentist.

8. A system as defined in claim 4 wherein said system includes program and data processing equipment for displaying (48) the packaging for a plurality of pharmaceutical products.

9. A system as defined in claim 4 wherein arrangements are provided for updating protocol information at said satellite installations (82, 94, 96) by information from the central server (98).

10. A system as defined in claim 4 wherein arrangements are provided for displaying radiology information on said monitor.

11. A system as defined in claim 4 wherein program and computer arrangements are provided for displaying prescription information (46) on said monitor and for printing out prescriptions.

12. A method for providing assistance to a dental surgeon while performing a dental operation, which comprises the steps of:
establishing a list (16) of various dental operations likely to be performed by the dental surgeon;
associating with each dental operation a clinical protocol (44) to be followed; and
displaying on a video monitor (90) upon the dental surgeon's demand, the said clinical protocol.

13. A method as defined in claim 12, **characterized in that** the displaying of the clinical protocol is accomplished by means of a sequence of illustrated steps, image by image.

14. A method as defined in claim 12, **characterized in that** the displaying of the clinical protocol is accomplished by means of dynamic animation (24).

15. A method as defined in claim 12, **characterized in that** it comprises in addition a step of displaying pharmaceutical products (48).

16. A method as defined in claim 15, **characterized in that** the displaying of pharmaceutical products is accomplished by means of visual identifiers which are specifically related to each product.

17. A method as defined in claim 12, **characterized in that** the clinical protocol related to the various dental operations are automatically updated by means of a connection to a central server (98) via an electronic data network (106).

18. A method as defined in claim 12 wherein a video monitor (90) is provided to display either patient history, a dental protocol or dental pharmaceutical information.

19. A method as defined in claims 12 including connecting a plurality of computers (92) and video monitors (90) at a plurality of dental offices (82, 94, 96) to a central server (98).

20. A method as defined in claim 19 further comprising associating a printer with each computer (92), and providing program and computer equipment to print out prescriptions from said printers.
